# EUROPEAN PATENT APPLICATION

(11) **EP 2 720 479 A2**
(43) Date of publication of application: **16.04.2014**
(21) Application number: 13187879.5
(22) Date of filing: 09.10.2013
(51) Int. Cl.: H04R 25/00

(54) **Cover for magnetic implant in a bone conduction hearing aid system and corresponding devices**

(30) Priority: 11.10.2012 US 201213650026; 11.10.2012 US 201213650057; 11.10.2012 US 201213649934; 11.10.2012 US 201213650080; 11.03.2013 US 201313793218
(71) Applicant: Sophono, Inc., Boulder, CO 80301 (US)
(72) Inventor: Kasic, James F., Boulder, CO Colorado 80303 (US); Haller, Markus C., 1196 Gland (CH); Siegert, Ralf, 45699 Herten (DE); Tautz, Margaret A., Littleton, CO Colorado 80123 (US)
(74) Representative: Lecomte & Partners

(57) **Abstract**

An implantable cover (200) configured to eliminate the need to form a recess in the skull (70) and to operate in conjunction with a magnetic implant (20) in a bone conduction hearing aid system (10). According to some embodiments, the cover (200) is configured and shaped to minimize patient discomfort and increase the positionability of the magnetic implant (20) on the patient's skull (70) when the magnetic implant (20) and the cover (200) are together implanted beneath the patient's skin (75), the cover (200) is disposed over at least portions of the magnetic implant (20), and the magnetic implant (20 is affixed to the patient's skull (70).

## Description

### Field of the Invention

Various embodiments of the invention described herein relate to the field of systems, devices, components, and methods for bone conduction hearing aid devices.

### Background

A magnetic bone conduction hearing aid is held in position on a patient's head by means of magnetic attraction that occurs between magnetic members included in the hearing aid and in a magnetic implant that has been implanted beneath the patient's skin and affixed to the patient's skull. Oftentimes, a magnetic implant may be affixed to the patient's skull in only one or two locations. If the patient's skin or tissue at such locations is particularly thin, if the patient's skin becomes irritated or inflamed while the magnetic hearing aid is being worn, or if the patent is uncomfortable or experiences discomfort or pain when wearing the hearing aid, then the only effective remedy may be to remove the magnetic hearing aid and the magnetic implant from the patient's head, as repositioning the magnetic hearing aid or the magnetic implant to a different location where good magnetic coupling and wearer comfort can still be achieved is not possible.

What is needed is a magnetic hearing aid and corresponding magnetic implant that somehow increase patient comfort and the number of positions in which a hearing aid can be located on the patient's head.

### Summary of the invention

According to a first aspect of the invention, there is provided a magnetic hearing system, comprising an electromagnetic ("EM") transducer, a magnetic spacer comprising at least first and second magnetic members, the magnetic spacer being configured to be mechanically and acoustically coupled to the EM transducer, a magnetic implant comprising at least third and fourth magnetic members, the magnetic implant being configured for implantation beneath a patient's skin and affixation to the patient's skull, and an implantable cover for the magnetic implant, the cover being configured for implantation beneath the patient's skin and in conformable contact and over at least portions of the magnetic implant.

According to a further aspect of the invention, there is provided an implantable cover for a magnetic implant, the cover and magnetic implant being configured for use in conjunction with a magnetic hearing device comprising an electromagnetic ("EM") transducer and a magnetic spacer comprising at least first and second magnetic members, the magnetic spacer being configured to be mechanically and acoustically coupled to the EM transducer, wherein the magnetic implant comprises at least third and fourth magnetic members configured to magnetically couple to the first and second magnetic members, the magnetic implant is configured for implantation beneath a patient's skin and affixation to the patient's skull, and the cover is configured for implantation beneath the patient's skin and in conformable contact with and over at least portions of the magnetic implant.

The cover may preferably comprise inner central portions and outer peripheral portions. The inner central portions may have at least a first thickness, the outer peripheral portions may have at least a second thickness, and the first thickness may exceed the second thickness. Preferably, the first thickness may range between about 1 mm and about 3 mm and the second thickness may range between about 0.2 mm and about 1.5 mm.

At least one tapered edge may preferably be disposed between the inner central portions and the outer peripheral portions of the cover.

The at least one tapered edge may preferably comprise a radius of curvature extending between the inner central portions and the outer peripheral portions that ranges between about 1 mm and about 10 mm, or between about 3 mm and about 7 mm.

Preferably, the cover may further be configured and shaped to minimize patient discomfort and increase the positionability of the magnetic implant on the patient's skull when the magnetic implant and the cover are together implanted beneath the patient's skin, the cover is disposed over and in conformable contact with at least portions of the magnetic implant, and the magnetic implant is affixed to the patient's skull.

The cover may further comprise at least one recess or hole or a matrix of tissue in-growth recesses or holes disposed therein or therethrough configured to permit the in-growth of tissue therethrough or therein.

The matrix may preferably be disposed in at least one wing attached to or forming a portion of the cover.

The cover may further comprise at least two recesses configured to accept the third and fourth magnetic members therein or therethrough.

Preferably, the cover may further be configured to permit the magnetic implant to be affixed to a patient's skull without the need to form a recess in the patient's skull configured to receive at least portions of the magnetic implant therein.

A plurality of recesses may preferably be formed in outer peripheral portions of the cover. The recesses may be configured to accept portions of a frame of the magnetic implant therein or therethrough.

At least portions of the cover may preferably be pliable, bendable, foldable, or malleable.

Preferably, the cover may further rounded or smoothed contours disposed on at least one of a top surface thereof, a side surface thereof, and a tapered edge thereof. The cover may comprise a bottom surface that is substantially flat.

Further embodiments are disclosed herein or will become apparent to those skilled in the art after having read and understood the specification and drawings hereof.

### Brief Description of the Drawings

Different aspects of the various embodiments will become apparent from the following specification, drawings and claims in which:
Figs. 1(a), 1(b) and 1(c) show side cross-sectional schematic views of selected embodiments of prior art SOPHONO ALPHA 1, BAHA and AUDIANT bone conduction hearing aids, respectively;
Fig. 2(a) shows one embodiment of a prior art functional electronic and electrical block diagram of hearing aid 10 shown in Figs. 1(a) and 3(b);
Fig. 2(b) shows one embodiment of a prior art wiring diagram for a SOPHONO ALPHA 1 hearing aid manufactured using an SA3286 DSP;
Fig. 3(a) shows one embodiment of prior art magnetic implant 20 according to Fig. 1(a), and various positions that overlying magnetic spacer 50 may assume in respect thereof;
Fig. 3(b) shows one embodiment of a prior art SOPHONO® ALPHA 1® hearing aid 10;
Figs. 4(a) through 4(f) show various views of one embodiment of magnetic implant 20 and corresponding implantable cover 200;
Figs. 4(c) and 4(d) show another embodiment of magnetic implant 20 and corresponding magnetic spacer 50;
Fig. 5 shows a side view of one embodiment of implantable cover 200 implanted beneath patient's skin 75 and in conformable contact with and over magnetic implant 20;
Fig. 6 shows one embodiment of a method 400 for implanting implantable cover 200 and magnetic implant 20 in a patient, and
Figs. 7(a) through 7(e) show further details regarding some of the steps described in connection with Fig. 6.

The drawings are not necessarily to scale. Like numbers refer to like parts or steps throughout the drawings.

### Detailed Description

Described herein are various embodiments of systems, devices, components and methods for bone conduction and/or bone-anchored hearing aids.

A bone-anchored hearing device (or "BAHD") is an auditory prosthetic device based on bone conduction having a portion or portions thereof which are surgically implanted. A BAHD uses the bones of the skull as pathways for sound to travel to a patient's inner ear. For people with conductive hearing loss, a BAHD bypasses the external auditory canal and middle ear, and stimulates the still-functioning cochlea via an implanted metal post. For patients with unilateral hearing loss, a BAHD uses the skull to conduct the sound from the deaf side to the side with the functioning cochlea. In most BAHA systems, a titanium post or plate is surgically embedded into the skull with a small abutment extending through and exposed outside the patient's skin. A BAHD sound processor attaches to the abutment and transmits sound vibrations through the external abutment to the implant. The implant vibrates the skull and inner ear, which stimulates the nerve fibers of the inner ear, allowing hearing. A BAHD device can also be connected to an FM system or iPod by means of attaching a miniaturized FM receiver or Bluetooth connection thereto.

BAHD devices manufactured by COCHLEAR™ of Sydney, Australia, and OPTICON™ of Smoerum, Sweden. SOPHONO™ of Boulder, Colorado manufactures an Alpha 1 magnetic hearing aid device, which attaches by magnetic means behind a patient's ear to the patient's skull by coupling to a magnetic or magnetized bone plate (or "magnetic implant") implanted in the patient's skull beneath the skin.

Surgical procedures for implanting such posts or plates are relatively straightforward, and are well known to those skilled in the art. See, for example, "Alpha I (S) & Alpha I (M) Physician Manual - REV A S0300-00" published by Sophono, Inc. of Boulder, Colorado, the entirety of which is hereby incorporated by reference herein.

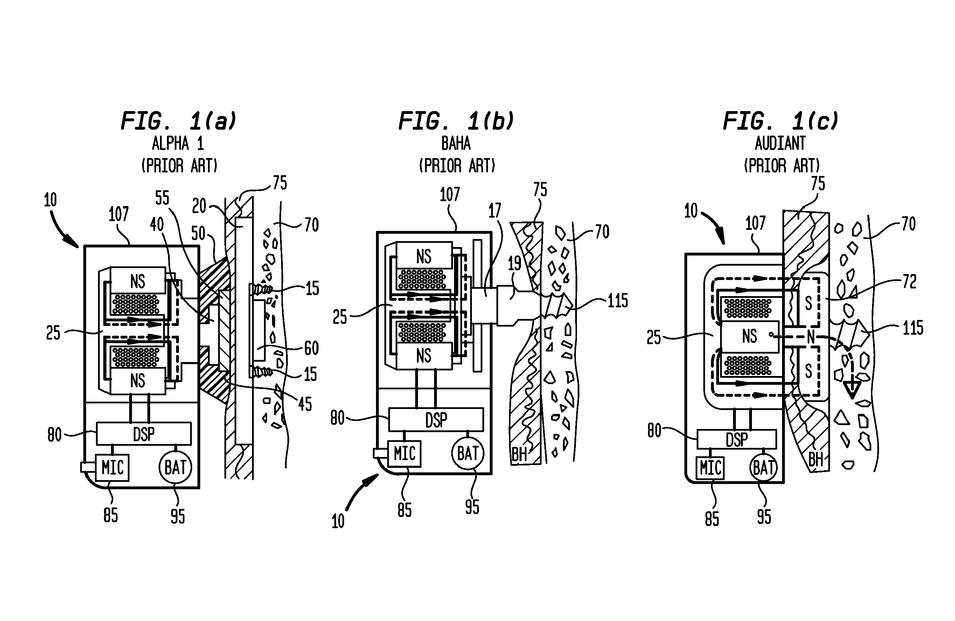

Figs. 1(a), 1(b) and 1(c) show side cross-sectional schematic views of selected embodiments of prior art SOPHONO ALPHA 1, BAHA and AUDIANT bone conduction hearing aids, respectively. Note that Figs. 1(a), 1(b) and 1(c) are not necessarily to scale.

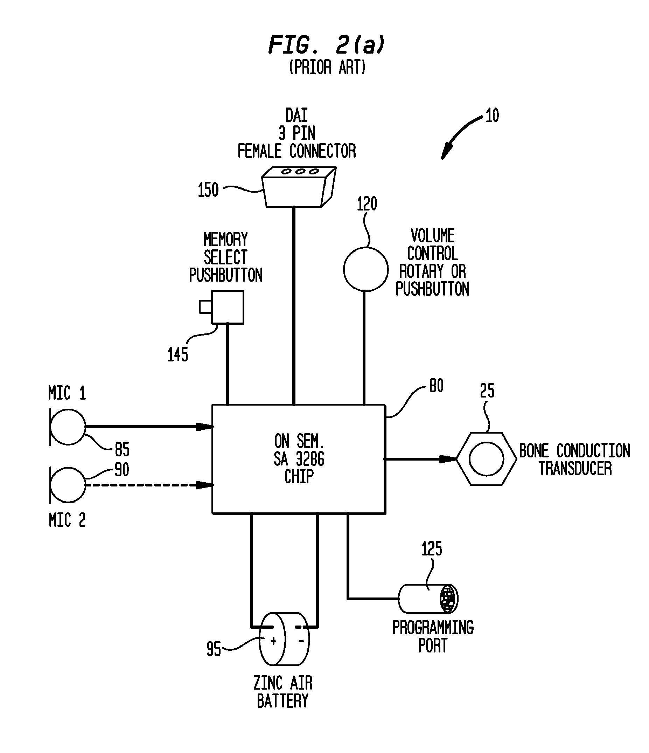

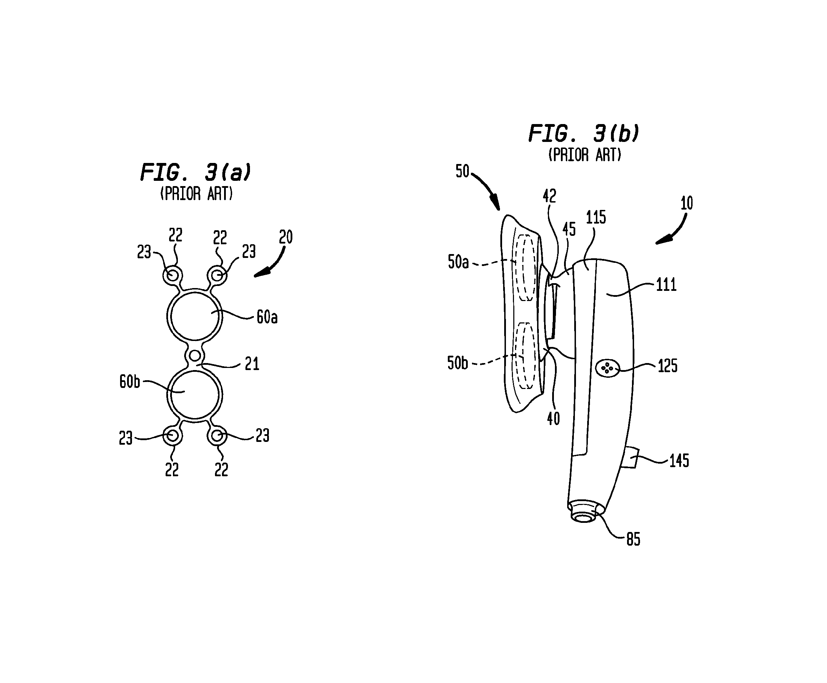

In Fig. 1(a), magnetic hearing aid device 10 comprises housing 107, electromagnetic/bone conduction ("EM") transducer 25 with corresponding magnets and coils, digital signal processor ("DSP") 80, battery 95, magnetic spacer 50, magnetic implant or magnetic implant bone plate 20. As shown in Figs. 1(a) and 2(a), and according to one embodiment, magnetic implant 20 comprises a frame 21 (see Fig. 3(a)) formed of a biocompatible metal such as medical grade titanium that is configured to have disposed therein or have attached thereto implantable magnets or magnetic members 60. Bone screws 15 secure or affix magnetic implant 20 to skull 70, and are disposed through screw holes 23 positioned at the outward ends of of arms 22 of magnetic implant frame 21 (see Fig. 2(a)). Magnetic members 60a and 60b are configured to couple magnetically to one or more corresponding external magnetic members or magnets 55 mounted onto or into, or otherwise forming a portion of, magnetic spacer 50, which in turn is operably coupled to EM transducer 25 and metal disc 40. DSP 80 is configured to drive EM transducer 25, metal disk 40 and magnetic spacer 50 in accordance with external audio signals picked up by microphone 85. DSP 80 and EM transducer 25 are powered by battery 95, which according to one embodiment may be a zinc-air battery, or may be any other suitable type of primary or secondary (i.e., rechargeable) electrochemical cell such as an alkaline or lithium battery.

As further shown in Fig. 1(a), magnetic implant 20 is attached to patient's skull 70, and is separated from magnetic spacer 50 by patient's skin 75. Hearing aid device 10 of Fig. 1(a) is thereby operably coupled magnetically and mechanically to plate 20 implanted in patient's skull 70, which permits the transmission of audio signals originating in DSP 80 and EM transducer 25 to the patient's inner ear via skull 70.

Fig. 1(b) shows another embodiment of hearing aid 10, which is a BAHA® device comprising housing 107, EM transducer 25 with corresponding magnets and coils, DSP 80, battery 95, external post 17, internal bone anchor 115, and abutment member 19. In one embodiment, and as shown in Fig. 1(b), internal bone anchor 115 includes a bone screw formed of a biocompatible metal such as titanium that is configured to have disposed thereon or have attached thereto abutment member 19, which in turn may be configured to mate mechanically or magnetically with external post 17, which in turn is operably coupled to EM transducer 25. DSP 80 is configured to drive EM transducer 25 and external post 17 in accordance with external audio signals picked up by microphone 85. DSP 80 and EM transducer 25 are powered by battery 95, which according to one embodiment is a zinc-air battery (or any other suitable battery or electrochemical cell as described above). As shown in Fig. 1(b), implantable bone anchor 115 is attached to patient's skull 70, and is also attached to external post 17 through abutment member 19, either mechanically or by magnetic means. Hearing aid device 10 of Fig. 1(b) is thus coupled magnetically and/or mechanically to bone anchor 15 implanted in patient's skull 70, thereby permitting the transmission of audio signals originating in DSP 80 and EM transducer 25 to the patient's inner ear via skull 70.

Fig. 1(c) shows another embodiment of hearing aid 10, which is an AUDIANT®-type device, where an implantable magnetic member 72 is attached by means of bone anchor 115 to patient's skull 70. Internal bone anchor 115 includes a bone screw formed of a biocompatible metal such as titanium, and has disposed thereon or attached thereto implantable magnetic member 72, which couples magnetically through patient's skin 75 to EM transducer 25. DSP 80 is configured to drive EM transducer 25 in accordance with external audio signals picked up by microphone 85. Hearing aid device 10 of Fig. 1(c) is thus coupled magnetically to bone anchor 15 implanted in patient's skull 70, thereby permitting the transmission of audio signals originating in DSP 80 and EM transducer 25 to the patient's inner ear via skull 70.

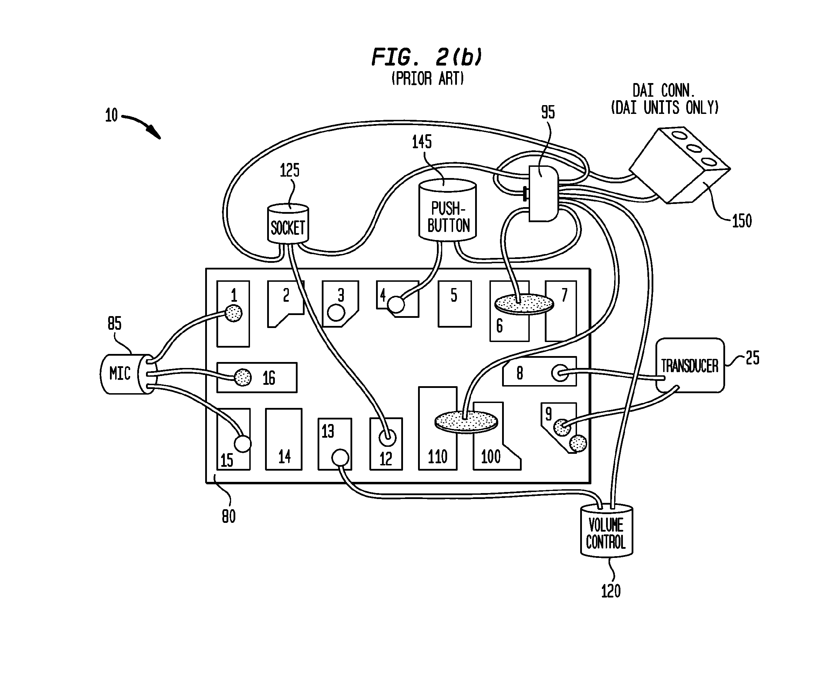

Fig. 2(a) shows one embodiment of a prior art functional electronic and electrical block diagram of hearing aid 10 shown in Figs. 1(a) and 2(b). In the block diagram of Fig. 2(a), and according to one embodiment, DSP 80 is a SOUND DESIGN TECHNOLOGIES® SA3286 INSPIRA EXTREME® DIGITAL DSP, for which data sheet 48550-2 dated March 2009, filed on even date herewith in an accompanying Information Disclosure Statement ("IDS"), is hereby incorporated by reference herein in its entirety. The audio processor for the SOPHONO ALPHA 1 hearing aid is centered around DSP chip 80, which provides programmable signal processing. The signal processing may be customized by computer software which communicates with the Alpha through programming port 125. According to one embodiment, the system is powered by a standard zinc air battery 95 (i.e. hearing aid battery), although other types of batteries may be employed. The SOPHONO ALPHA 1 hearing aid detects acoustic signals using a miniature microphone 85. A second microphone 90 may also be employed, as shown in Fig. 2(a). The SA 3286 chip supports directional audio processing with second microphone 90 to enable directional processing. Direct Audio Input (DAI) connector 150 allows connection of accessories which provide an audio signal in addition to or in lieu of the microphone signal. The most common usage of the DAI connector is FM systems. The FM receiver may be plugged into DAI connector 150. Such an FM transmitter can be worn, for example, by a teacher in a classroom to ensure the teacher is heard clearly by a student wearing hearing aid 10. Other DAI accessories include an adapter for a music player, a telecoil, or a Bluetooth phone accessory. According to one embodiment, DSP 80 or SA 3286 has 4 available program memories, allowing a hearing health professional to customize each of 4 programs for different listening situations. The Memory Select Pushbutton 145 allows the user to choose from the activated memories. This might include special frequency adjustments for noisy situations, or a program which is Directional, or a program which uses the DAI input.

Fig. 2(b) shows one embodiment of a prior art wiring diagram for a SOPHONO ALPHA 1 hearing aid manufactured using the foregoing SA3286 DSP. Note that the various embodiments of hearing aid 10 are not limited to the use of a SA3286 DSP, and that any other suitable CPU, processor, controller or computing device may be used. According to one embodiment, DSP 80 is mounted on a printed circuit board 155 disposed within housing 110 and /or housing 115 of hearing aid 10 (not shown in the Figures).

In some embodiments, the microphone incorporated into hearing aid 10 is an 8010T microphone manufactured by SONION®, for which data sheet 3800-3016007, Version 1 dated December, 2007, filed on even date herewith in the accompanying IDS, is hereby incorporated by reference herein in its entirety. Other suitable types of microphones, including other types of capacitive microphones, may be employed.

In still further embodiments, the electromagnetic transducer 25 incorporated into hearing aid 10 is a VKH3391W transducer manufactured by BMH-Tech® of Austria, for which the data sheet filed on even date herewith in the accompanying IDS is hereby incorporated by reference herein in its entirety. Other types of suitable EM transducers may also be used.

Figs. 3(a) and 3(b) show implantable bone plate or magnetic implant 20 in accordance with Fig. 1(a), where frame 22 has disposed thereon or therein magnetic members 60a and 60b, and where magnetic spacer 50 of hearing aid 10 has magnetic members 55a and 55b spacer disposed therein. The two magnets 60a and 60b of magnetic implant 20 of Fig. 2(a) permit hearing aid 10 and magnetic spacer 50 to be placed in a single position on patient's skull 70, with respective opposing north and south poles of magnetic members 55a, 60a, 55b and 60b appropriately aligned with respect to one another to permit a sufficient degree of magnetic coupling to be achieved between magnetic spacer 50 and magnetic implant 20 (see also Fig. 3(b)). As shown in Fig. 1(a), magnetic implant 20 is preferably configured to be affixed to skull 70 under patient's skin 75. In one aspect, affixation of magnetic implant 20 to skull 75 is by direct means, such as by screws 15. Other means of attachment known to those skilled in the art are also contemplated, however, such as glue, epoxy, and sutures.

Referring now to Fig. 3(b), there is shown a SOPHONO® ALPHA 1® hearing aid 10 configured to operate in accordance with magnetic implant 20 of Fig. 3(a). As shown, hearing aid 10 of Fig. 3(b) comprises upper housing 111, lower housing 115, magnetic spacer 50, external magnets 55a and 55b disposed within spacer 50, EM transducer diaphragm 45, metal disk 40 connecting EM transducer 25 to spacer 50, programming port/socket 125, program switch 145, and microphone 85. Not shown in Fig. 3(b) are other aspects of the embodiment of hearing aid 10, such as volume control 120, battery compartment 130, battery door 135, battery contacts 140, direct audio input (DAI) 150, and hearing aid circuit board 155 upon which various components are mounted, such as DSP 80.

Continuing to refer to Figs. 3(a) and 3(b), frame 22 of magnetic implant 20 holds a pair of magnets 60a and 60b that correspond to magnets 55a and 55b included in spacer 50 shown in Fig. 3(b). The south (S) pole and north (N) poles of magnets 55a and 55b, are respectively configured in spacer 50 such that the south pole of magnet 55a is intended to overlie and magnetically couple to the north pole of magnet 60a, and such that the north pole of magnet 55b is intended to overlie and magnetically couple to the south pole of magnet 60b. This arrangement and configuration of magnets 55a, 55b, 60a and 60b is intended permit the magnetic forces required to hold hearing aid 10 onto a patient's head to be spread out or dispersed over a relatively wide surface area of the patient's hair and/or skin 75, and thereby prevent irritation of soreness that might otherwise occur if such magnetic forces were spread out over a smaller or more narrow surface area. In the embodiment shown in Fig. 3(a), frame 22 and magnetic implant 20 are configured for affixation to patient's skull 70 by means of screws 15, which are placed through screw recesses or holes 23.

Still referring to Figs. 3(a) and 3(b), during use or wearing of hearing aid 10 thereof and positioning of same over magnetic implant 20, it has been discovered that despite the magnetic-force-spreading intent of the design illustrated in Figs. 3(a) and 3(b), skin soreness and irritation may still occur in some patients. As mentioned above, magnetic implant 20 may typically be affixed to patient's skull 70 in a limited number of locations, such as only one or two locations. If the patient's skin or tissue 75 at such locations is particularly thin, if the patient's skin becomes irritated or inflamed while the magnetic hearing aid 10 is being worn, or if the patent is uncomfortable or experiences discomfort or pain when wearing the hearing aid 10, then the only effective remedy may be to remove the magnetic implant 20 from the patient's head, as repositioning the magnetic implant 20 in a different location where good magnetic coupling and wearer comfort can still be achieved may not be possible.

What is needed is a magnetic hearing aid 10 and corresponding magnetic implant 20 that somehow increase patient comfort and the number of positions in which a hearing aid 10 can be located on the patient's head, yet that still provides the required amount of magnetic force and coupling to hold hearing aid 10 on patient's skull 70 during actual use.

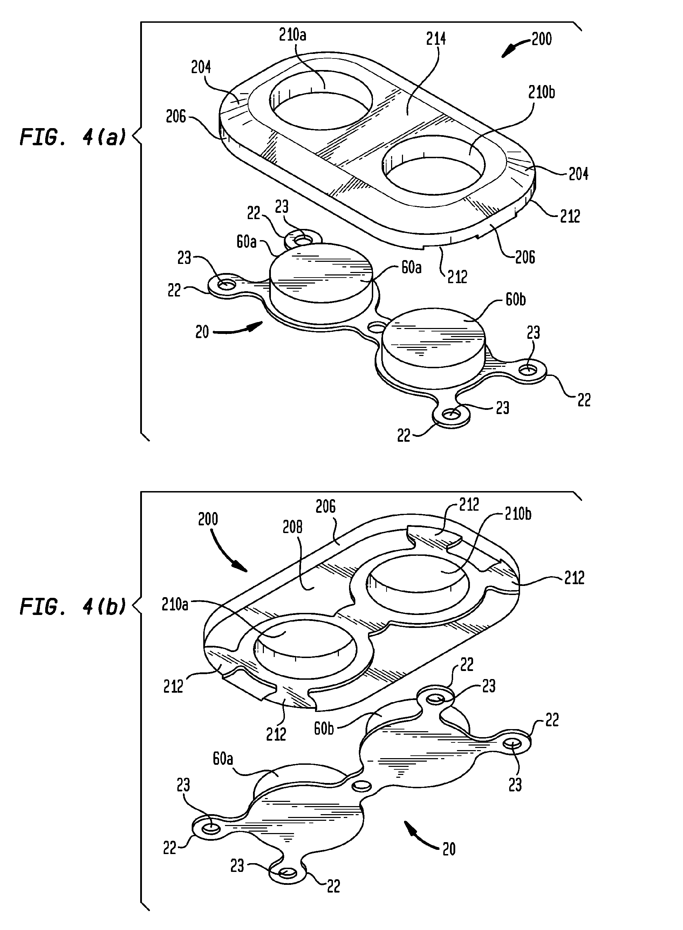

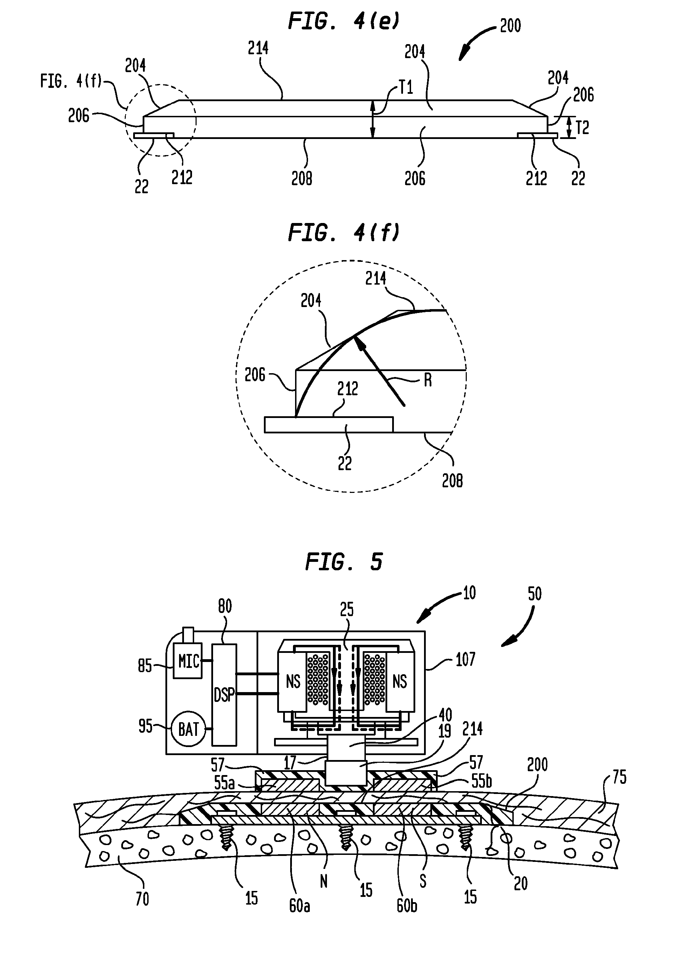

Referring now to Figs. 4(a) through 4(f), there are shown various views of one embodiment of magnetic implant 20 and corresponding implantable cover 200, which are configured such that magnetic implant 20 may be affixed to patient's skull 70 beneath patients skin 75 with cover 200 in conformable contact therewith, and with cover 200 disposed over at least portions of magnetic implant 20. According to some embodiments, cover 200 is further configured and shaped to minimize patient discomfort and increase the positionability of magnetic implant 20 on patient's skull 70 when magnetic implant 20 and cover 200 are together implanted beneath the patient's skin, more about which is said below.

Fig. 4(a) shows a top perspective view of one embodiment of cover 200 and magnetic implant 20. Cover 200 comprises recesses 210a and 210b configured to receive therein magnetic members 60a and 60b when cover 200 is placed in conformable contact with and over implant 20. Recesses 212 formed along the bottom edges of cover 200 are configured to permit protruding portions of frame 22 in magnetic implant 20 to be received and fit therein. The dimensions of cover 200 may be selected such that screw holes 23 may protrude completely, partially or not at all from beneath cover 200 when cover 200 is placed over and in conformable contact with implant 20.

As further shown in Fig. 4(a), cover 200 comprises top surface 214, tapered edge 204 and side edge 206. As a result, the thickness of cover 200 is reduced around the outer periphery thereof with respect to central inner portions thereof. Cover 200 thus does not present the abrupt edge transitions that magnetic implant 20 alone otherwise would, which have been discovered to be one of the sources of the patient discomfort, irritation and pain described above. When implant 20 has been affixed to patient's skull 70, and cover 20 has been placed thereover in conformable contact therewith beneath patient's skin 75, the abrupt edge transitions otherwise presented by magnetic implant 20 are minimized or eliminated. As a result, no recess needs to be formed in patient's skull 70 to receive magnetic implant 20 therein, which is another, albeit much more time-consuming, way to reduce or eliminate abrupt edge transitions caused when magnetic implant 20 is implanted beneath patient's skin 75. The rounded edges of cover 200 further reduce patient discomfort, irritation and pain. Cover 20 also permits an increased number of locations on a patient's skull 70 to be utilized as potential magnetic implant affixation sites owing to the reduction of patient discomfort, irritation and pain resulting from its rounded contours and thinner edges.

Fig. 4(b) shows a bottom perspective view of cover 200 and magnetic implant 20 shown in Fig. 4(a). Cover 200 comprises recesses 210a and 210b configured to receive therein magnetic members 60a and 60b when cover 200 is placed in conformable contact with and over implant 20. Recesses 212 are formed along the bottom edges of cover 200, and are configured to permit protruding portions of frame 22 in magnetic implant 20 to be received and fit therein. As further shown in Fig. 4(b), cover 200 comprises bottom surface 208 and side edge 206.

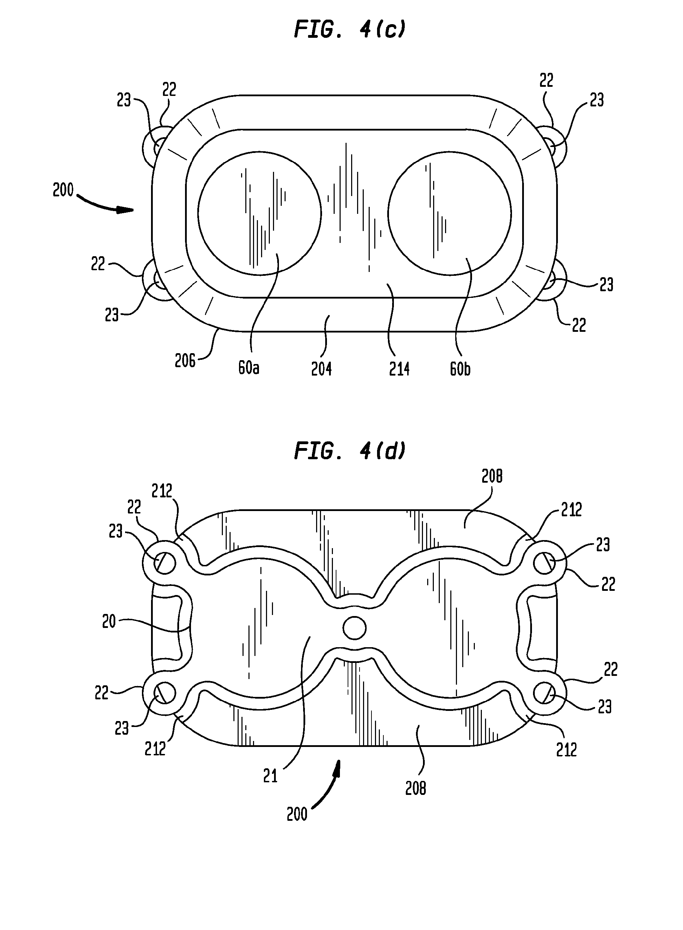

Figs. 4(c) and 4(d) show top and bottom view of the embodiment of cover 200 shown in Figs. 4(a) and 4(b). Fig. 4(e) shows a side view of the embodiment of cover 200 shown in Figs. 4(a) through 4(d), and illustrates how first thickness T1 of cover 200 at central inner portions thereof exceeds that of second thickness T2 at outer peripheral portions thereof. According to some embodiments, the first thickness may range between about 1 mm and about 3 mm, and the second thickness may range between about 0.2 mm and about 1.5 mm. One preferred thickness for the first thickness is about 2 mm. A preferred thickness for the second thickness is about 0.5 mm. Other ranges of thickness, and thicknesses, are contemplated.

As shown in Figs. 4(a) through 4(e), at least one tapered edge 204 may be disposed between the inner central portions and the outer peripheral portions of cover 200. As further shown in Fig. 4(e), and according to some embodiments, tapered edge 204 may be characterized by a radius of curvature R that extends between the inner central portions and the outer peripheral portions of cover 200 that ranges between about 1 mm and about 10 mm, or between about 3 mm and about 7 mm. Other ranges of radii, and other radii, are also contemplated, such as greater than 50mm, greater than 75mm, and greater than 100 mm. Cover 200 may comprise rounded or smoothed contours disposed on at least one of a top surface 214 thereof, a bottom surface 208 thereof, a side surface 206 thereof, and/or a tapered edge 204 thereof. As shown in the embodiments illustrated in Figs. 4(a) through 5, cover 200 comprises a bottom surface 208 that is substantially flat.

Fig. 5 shows a side view of one embodiment of implantable cover 200 implanted beneath patient's skin 75 and in conformable contact with and over magnetic implant 20, which has been affixed to patient's skull 70 by means of screws 15. Note that no recess has been formed in skull 70 to receive magnetic implant 20 therein. Cover 20 eliminates the need to form such a recess since the abrupt edge transitions presented by magnetic implant 20 are eliminated by placing cover 200 thereover and in conformable contact therewith. By forming cover 200 from a malleable, flexible or at least somewhat elastic or deformable material, the edge-reducing or minimizing effects of cover 200 can be further enhanced.

Referring now to Figs. 4(a) through 5, it will be seen that cover 200 may be configured and shaped to minimize patient discomfort and increase the positionability of magnetic implant 20 on patient's skull 70 when magnetic implant 20 and cover 200 are together implanted beneath patient's skin 75, cover 200 is disposed over and in conformable contact with at least portions of magnetic implant 20, and magnetic implant 20 is affixed to patient's skull 70.

Because cover 200 is implantable, cover 200 preferably comprises a biocompatible material such as a suitable metal or metal alloy, a polymer, a plastic, rubber, silicone, a fabric, an impregnated fabric, nylon, polyethylene terephthalate (PET), PTFE, PEEK, PMMA, polyethylene, polyester, synthetic fibers, a thermoplastic polymer, a bioresorbable polymer, a drug-loaded or filled polymer, a drug-eluting polymer, or a polymer comprising one or more bioactive materials. As noted above, cover 200 may also be formed of a material that is malleable, flexible or at least somewhat elastic or deformable, thereby enhancing the edge-reducing or minimizing effects of cover 200. Cover 200 may further be formed of a material that renders cover 200 pliable, bendable, foldable or malleable, thereby increasing the ease with which cover 200 may be implanted beneath a patient's skin 75, more about which is said below.

Cover 200 may further comprise at least one recess or hole configured to permit the in-growth of tissue therethrough or therein, or a matrix of tissue in-growth recesses or holes disposed therein or therethrough. Such a matrix may be disposed in at least one wing attached to or forming a portion of cover 200.

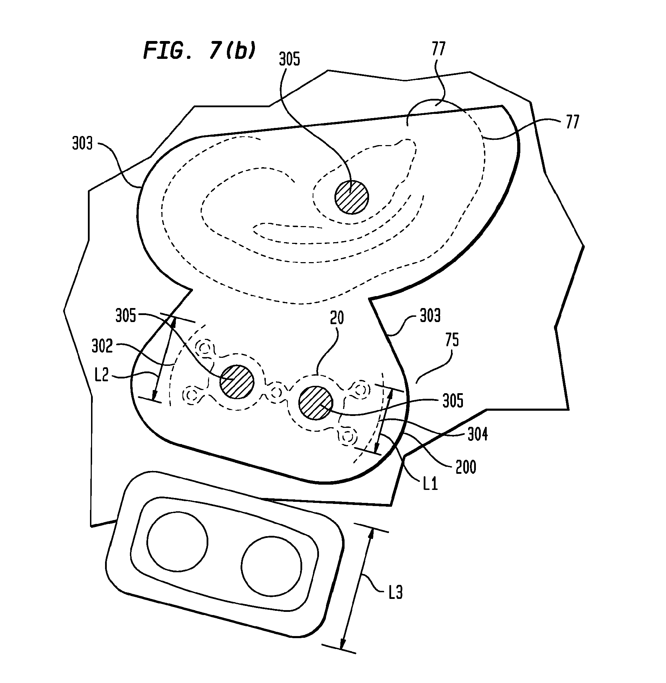

Continuing to refer to Figs. 4(a) through 5, and also now to Fig. 7(b), according to various embodiments cover 200 and magnetic implant 20 may be further configured such that arms 22 of magnetic implant 20 having screw or attachment holes or recesses 23 disposed therethrough are have a width L2 that is slightly greater than, or of approximately the same or close to the width L3 as, that of magnetic members 60a and 60b L2, thereby easing implantation of magnetic member 20 through incisions 302 and 304, more about which is said below. The smaller the footprint of magnetic implant 20and cover 200, and especially widths L1 and L3 thereof, the easier it is to surgically implant magnetic member 20 through incision 302 or incision 304.

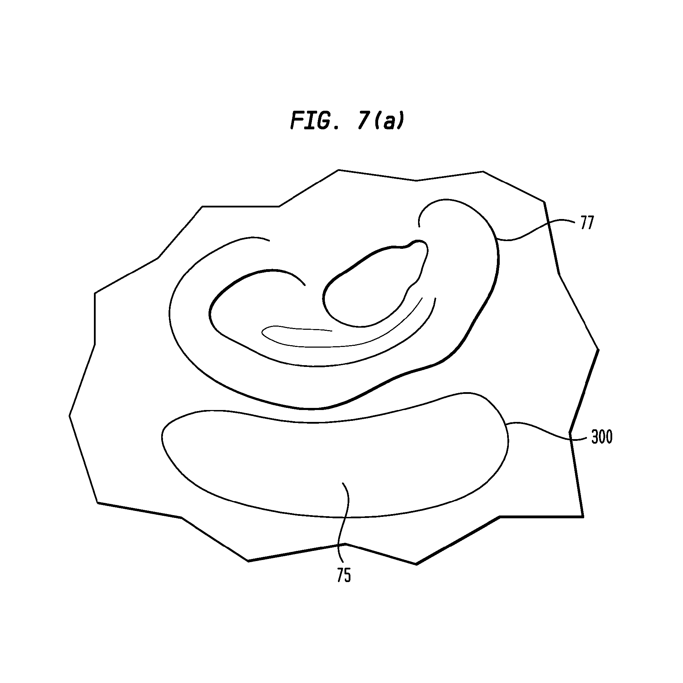

As discussed above, and also below in connection with Figs. 7(a) through 7(e), once magnetic implant 20 has been positioned by a surgeon in the correct or desired position, screws 15 are positioned through holes 23 to attach magnetic implant 20 to patient's skull 70 using a surgical screwdriver and other means and methods well understood by those practicing in the surgical implantation arts. In the embodiment shown in Figs. 4(a) through 7(e), four screws 15 are employed to attach magnetic implant 20 to patient's skull 70. Other numbers of screws 15, recesses or holes 23, and/or arms 22 of magnetic implant 20 are contemplated and fall within the scope of the present description and disclosure, as those skilled in the art will now understand after having read and understood the present specification and drawings. Moreover, cover 200 may be configured to permit or free access to screw holes 23 once cover 20 after cover 20 has been placed over magnetic implant 20. For example, cover 200 may be configured such that portions of its periphery do not cover or overlap screw holes 23. Cover 200 may also be formed of a flexible or malleable material that permits the edges or periphery thereof to be moved aside easily to permit access to screws 15 while magnetic implant 20 is being affixed to patient's skull 70 during the implant procedure.

Referring now to Figs. 4(a) through 4(d), in some embodiments, cover 200 is configured so that it forms an interference fit over magnetic implant 20. The material from which cover 200 is formed, and the dimensions of holes or recesses 210a and 210b, are configured such that cover 200 fits relatively tightly over magnetic members 60a and 60b when operably placed in position thereover. In such embodiments, the material from which cover 200 is formed is elastic or pliable in nature, and capable of stretching over and engaging tightly magnetic members 60a and 60b as cover 200 is placed operably in position over magnetic implant 20. Over 200 may also be configured such that it is stretchable along its width and/or length to facilitate proper placement over, and engagement with, magnetic implant 20. In still other embodiments, cover 200 and magnetic implant 20 are together configured such that when cover 200 is placed in an operable position over magnetic implant 20, detents, tangs, protrusions, tabs, channels and corresponding matable protrusions or other mechanical features or elements disposed on cover 200 and magnetic implant 20 grip and hold cover 200 onto magnetic implant 20. After having read and understood the present specification and drawings, those skilled in the art will now understand that many different permutations and combinations detents, tangs, protrusions, tabs, channels and corresponding matable protrusions or other mechanical features or elements are contemplated and fall within the scope of the present description and disclosure.

Cover 200 may also be formed of or include shape memory materials, such as shape memory polymers, plastics, thermoplastics, and/or metals to further facilitate proper positioning over and/or engagement with magnetic implant 20. Various types of adhesives may also be employed to secure or aid in securing cover 200 to magnetic implant 20, such as biocompatible epoxies, curable epoxies, silicone and other medical grade adhesives known in the art.

In further embodiments, cover 200 is configured such that it may be folded, rolled or otherwise have at least some of its dimensions reduced during implantation so that cover 200 may be inserted through incisions 302 and/or 304 more easily (see Figs. 7(a) through 7(e)), more about which is said below. Rolled or folded cover 200 may also be inserted through incisions 302 and/or 304 by means of a catheter or introducer. For example, rolled or folded cover 200 may be inserted in the distal end of a catheter or introducer, and then pushed out of the catheter through incision 302 and/304 during the implantation procedure by means of a plunger proximally located in the catheter and pushed in a distal direction. To facilitate placing cover 200 into a rolled or folded configuration, cover 200 may include one or more channels or recesses formed therein that are disposed along the folding axis, and which facilitate or ease bending or folding of cover 200. Such recesses or channels may be formed along the bottom or top surface of cover 200, or inside cover 200. Alternatively, and to the same end, cover 200 may be formed such that portions thereof disposed along the folding axis are more pliable or bendable material than other portions of cover 200. For example, such portions could include a honeycombed or lightened structure that facilitates bending or folding.

In still other embodiments, cover 200 may comprise separate or disparate pieces or components that may be assembled to form a complete cover before or after cover 200 has been implanted. For example, cover 200 may comprise a central portion that is configured to engage and fit over magnetic implant 20 and magnetic member 60a and 60b. Peripheral portions or wings may then be attached to the central portion of cover 200 once cover 200 has been placed in position over magnetic implant 20, or prior to implantation. The peripheral portions or wings may be configured to permit the size, shape and/or function of cover 200 to be customized by the surgeon according to a particular patient's needs or skull geometry. For example, one such peripheral portion or wing may be of lesser or greater width or length than the other peripheral portion to accommodate variations from the norm of a particular patient's skull geometry. Such peripheral portions or wings may also be configured to permit the in-growth of tissue therethrough (or not), or to permit replacement of such peripheral portion or wing at a later date with a peripheral portion or wing of different dimensions or other characteristics. Moreover, such peripheral portions or wings may be attached or secured to the central portion by any of a number of different means, such as medical grade adhesives, detents, tangs, protrusions, tabs, channels and corresponding matable protrusions or other mechanical features or elements, tape, or other mechanical components or devices.

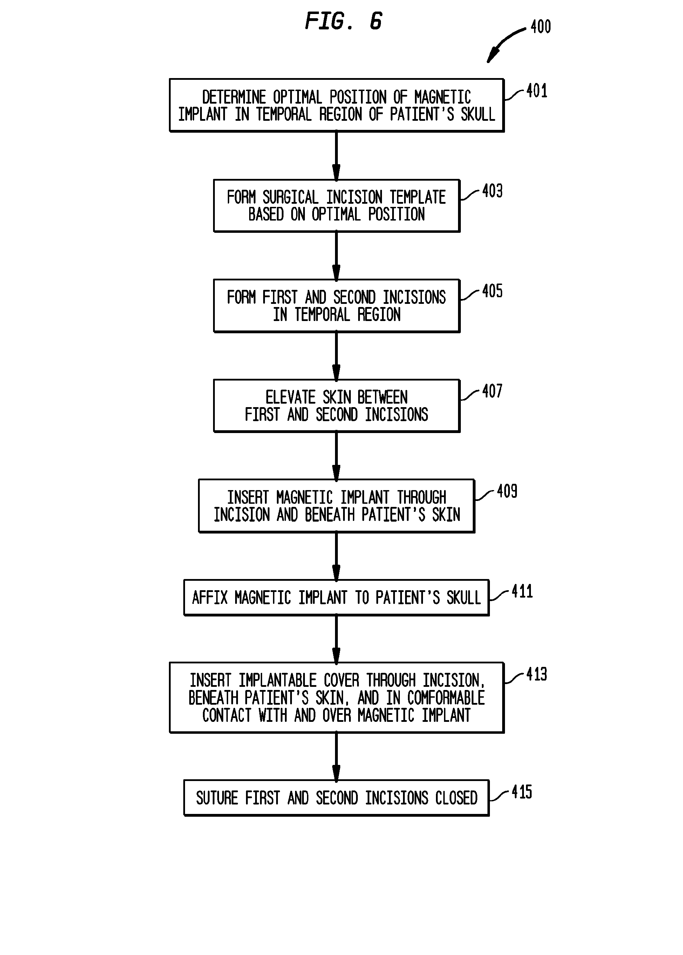

Turning now to Fig. 6, there is illustrated one embodiment of a method 400 for implanting implantable cover 200 and magnetic implant 20 in a patient. At step 401, an optimal position of magnetic implant 20 in the temporal region of a patient's skull 70 is determined behind the patient' s ear. Step 410 may be carried out by placing magnetic implant 200 over the temporal region and sliding it back and forth and around until a suitable or optimal position of magnetic implant 20 is determined, sensed or discovered by the physician or health care provider.

At step 403, a surgical incision template is formed based on the determined optimal position of magnetic implant 20. According to one embodiment, the determined optimal position is recorded or transcribed onto a clear plastic template onto which the physician or health care provider inscribes with a marker the relative positions of implant 20, the outlines of the outer portions of the patient's ear, the ear canal, and the surgical incision locations. The template can then be used later by the physician to accurately position magnetic implant 20 during the implantation procedure.

Next, at step 405, and after applying a local anesthetic to the temporal region behind the patient's ear, first and second incisions are formed through the patient's skin and down to the bone in the temporal region behind the patient's ear, preferably with the aid of the template that was previously formed. According to one embodiment, the first and second surgical incisions are separated by a distance of about 5 cm.

At step 407, the skin between the first and second incisions is lifted, preferably by sub-periosteal means. A rasp or other surgical tool may be employed to lift the skin between the incisions. At step 409, magnetic implant 20 is inserted through one of the two incisions and beneath patient's skin 75, followed at step 411 by affixation of magnetic implant 20 to the patient's skull 70. At step 413, implantable cover 200 is inserted through one of the two incisions beneath the patient's skin 75 and in conformable contact with and over magnetic implant 20. Lastly, at step 415 the first and second incisions are sutured closed. Absorbable sutures may be employed in step 415.

Further details regarding some of the steps described above in connection Fig. 6 are shown in Figs. 7(a) through 7(e). In Fig. 7(a), there is shown temporal region 300 behind patient's ear 77. Fig. 7(b) shows one embodiment of transparent or translucent template 303, which may be marked by a physician or health care provider with marks 305 to aid in the accurate positioning of the first and second incisions 302 and 304 (shown as dashed line marks on template 303), and to aid in the subsequent accurate positioning and affixation of magnetic implant 20 beneath the patient's skin and to the patient's skull. As shown, template 303 can be marked with dots 305 positioned over magnetic members 60a and 60b, and over the ear canal of the patient. Template 303 can also be marked to show where the outlines of the patient's ear lie, and where the first and second incisions should be made.

According to some embodiments, and as shown in Fig. 7(b), incision 302 and/304 has a width L2 that is slightly greater than, or about the same width L2 as, width L1 of magnetic implant 20, thereby facilitating insertion of magnetic implant 20 through incision 302 and/or 304 during the implantation procedure. As further shown in Fig. 7(b), and according to some embodiments, cover 200 has a width L3 that is greater than either or both of widths L1 and L2. As discussed above, cover 200 may be formed or a pliable, elastic, bendable or foldable material, and may be configured such that cover 200 can be bent or folded along one or more dimensions for insertion through smaller width incision 302 and/or 304 during the implantation procedure. Alternatively, and as discussed above, cover 200 may be configured and shaped for delivery atop and onto magnetic implant 20 by means of a catheter or introducer through incision 302 and/or 304. In either case, one goal is to make incisions 302 and 304 as small as possible, thereby reducing patient trauma and improving healing time.

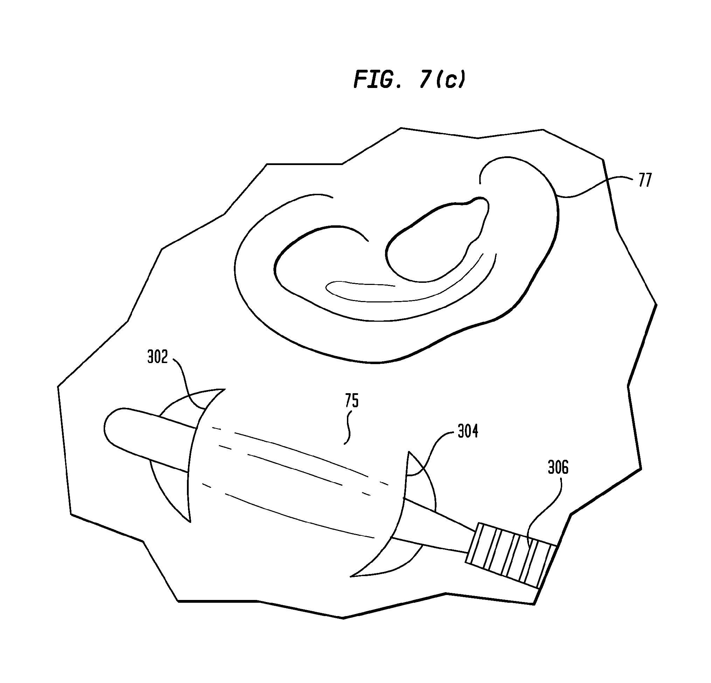

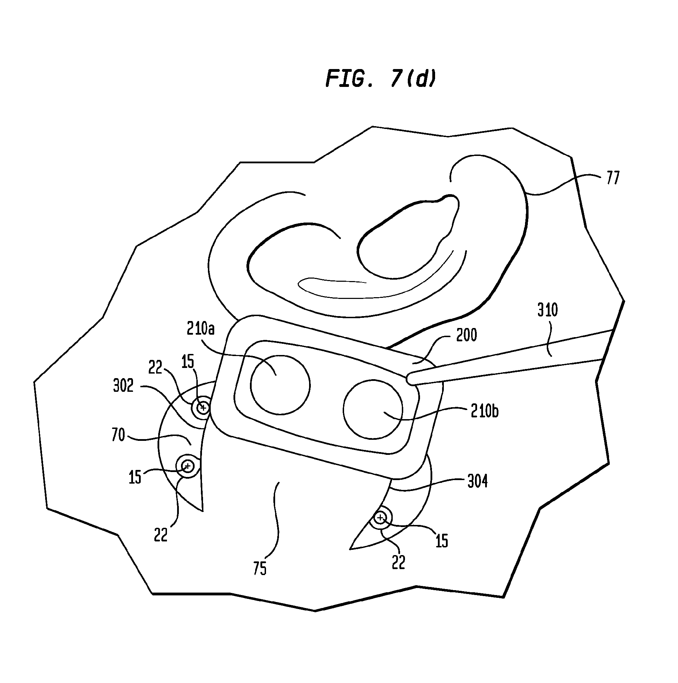

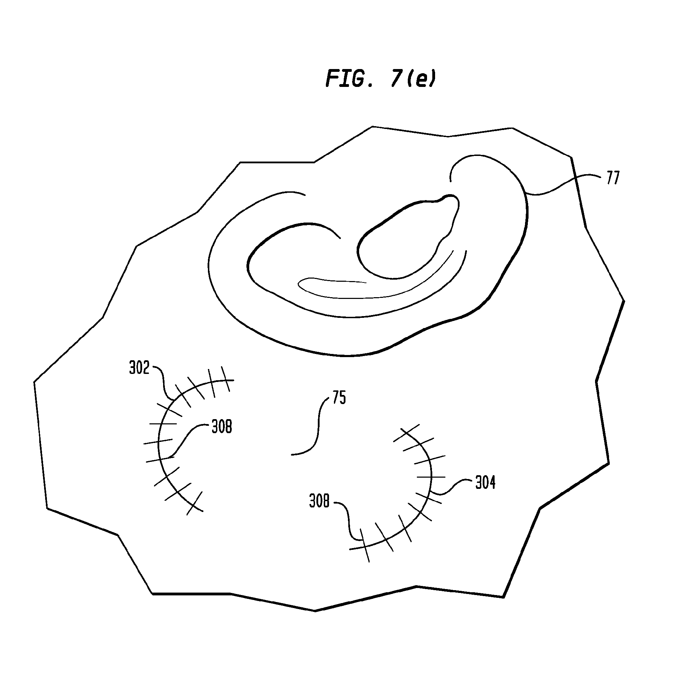

Fig. 7(c) shows rasp 306 in use to lift skin between incisions 302 and 304. Fig. 7(d) shows magnetic implant 20 implanted beneath patient's skin 75 and affixed to patient's skull 70 by means of screws 15. Cover 200 is shown ready to be inserted through first incision 302 or second incision 304 by means of surgical tool or tweezers 310 for conformable contact and engagement with and over magnetic implant 20, and beneath patient's skin 75. In one embodiment, cover 200 is formed of a deformable, bendable or rollable material such that it may have a width greater than that of incision 302 or 304, and yet still be insertable therethrough for implantation atop and in contact with magnetic implant 20. Fig. 7(e) shows incisions 302 and 304 after they have been closed with sutures 308.

Several important results follow from the methods illustrated in Figs. 6 and 7(a) through 7(e). First, the described surgical techniques and corresponding devices are simple to employ. Second, they are quick; the procedures described , above in connection with Figs. 6 and 7(a) through 7(e) can be completed in as little as 15 minutes. Third, they are cost-effective, and can be carried out on an outpatient basis. Fourth, they permit early primary fitting of a bone conduction hearing aid on a patient. Fifth, they reduce patient discomfort, irritation and pain. Sixth, they expand the population of patients who can be successfully fitted with a bone conduction hearing aid.

Those skilled in the art will now understand that many different permutations, combinations and variations of magnetic implant 20 and implantable cover 200 fall within the scope of the various embodiments. For example, cover 200 may have a continuously reducing thickness towards its edges, or may have more or fewer recess 210a, 210b and 212 in cover 200. Surgical techniques other than those described or disclosed explicitly herein may be employed to implant magnetic implant 20 and cover 200. Those skilled in the art will now appreciate that many different combinations, permutations and configurations of magnetic implants and implantable covers may be employed to arrive at suitable configurations of same. Moreover, the above-described embodiments should be considered as examples, rather than as limiting the scopes thereof.

## Claims

1. A magnetic hearing system, comprising:
an electromagnetic ("EM") transducer;
a magnetic spacer comprising at least first and second magnetic members, the magnetic spacer being configured to be mechanically and acoustically coupled to the EM transducer;
a magnetic implant comprising at least third and fourth magnetic members, the magnetic implant being configured for implantation beneath a patient's skin and affixation to the patient's skull, and
an implantable cover for the magnetic implant, the cover being configured for implantation beneath the patient's skin and in conformable contact and over at least portions of the magnetic implant.

2. An implantable cover for a magnetic implant, the cover and magnetic implant being configured for use in conjunction with a magnetic hearing device comprising an electromagnetic ("EM") transducer and a magnetic spacer comprising at least first and second magnetic members, the magnetic spacer being configured to be mechanically and acoustically coupled to the EM transducer, wherein the magnetic implant comprises at least third and fourth magnetic members configured to magnetically couple to the first and second magnetic members, the magnetic implant is configured for implantation beneath a patient's skin and affixation to the patient's skull, and the cover is configured for implantation beneath the patient's skin and in conformable contact with and over at least portions of the magnetic implant.

3. The device of claim 1 or 2, wherein the cover comprises inner central portions and outer peripheral portions, the inner central portions have at least a first thickness, the outer peripheral portions have at least a second thickness, and the first thickness exceeds the second thickness.

4. The device of claim 3, wherein the first thickness ranges between about 1 mm and about 3 mm and wherein the second thickness ranges between about 0.2 mm and about 1.5 mm.

5. The device of any of claims 3 or 4, wherein at least one tapered edge is disposed between the inner central portions and the outer peripheral portions.

6. The device of claim 5, wherein the at least one tapered edge comprises a radius of curvature extending between the inner central portions and the outer peripheral portions that ranges between about 1 mm and about 10 mm, or between about 3 mm and about 7 mm.

7. The device of any of claims 1 to 6, wherein the cover is further configured and shaped to minimize patient discomfort and increase the positionability of the magnetic implant on the patient's skull when the magnetic implant and the cover are together implanted beneath the patient's skin, the cover is disposed over and in conformable contact with at least portions of the magnetic implant, and the magnetic implant is affixed to the patient's skull.

8. The device of any of claims 1 to 7, wherein the cover further comprises at least one recess or hole or a matrix of tissue in-growth recesses or holes disposed therein or therethrough configured to permit the in-growth of tissue therethrough or therein.

9. The device of claim 8, wherein the matrix is disposed in at least one wing attached to or forming a portion of the cover.

10. The device of any of claims 1 to 9, wherein the cover comprises at least two recesses configured to accept the third and fourth magnetic members therein or therethrough.

11. The device of any of claims 1 to 10, wherein the cover is configured to permit the magnetic implant to be affixed to a patient's skull without the need to form a recess in the patient's skull configured to receive at least portions of the magnetic implant therein.

12. The device of any of claims 1 to 11, further comprising a plurality of recesses formed in outer peripheral portions thereof that are configured to accept portions of a frame of the magnetic implant therein or therethrough.

13. The device of any of claims 1 to 12, wherein at least portions of the cover are pliable, bendable, foldable, or malleable.

14. The device of any of claims 1 to 13, wherein the cover comprises rounded or smoothed contours disposed on at least one of a top surface thereof, a side surface thereof, and a tapered edge thereof.

15. The device of any of claims 1 to 14, wherein the cover comprises a bottom surface that is substantially flat.
